# EUROPEAN PATENT APPLICATION

(11) **EP 2 009 113 A1**
(43) Date of publication of application: **31.12.2008**
(21) Application number: 07111167.8
(22) Date of filing: 27.06.2007
(51) Int. Cl.: C12Q 1/68

(54) **Fusion gene microarray**

(71) Applicant: Rikshospitalet- Radiumhospitalet HF, 0027 Oslo (NO)
(72) Inventor: Skotheim, Rolf I., 0372 Oslo (NO); Lothe, Ragnhild A., 0267 Oslo (NO); Lind, Guro E., 0770 Oslo (NO)
(74) Representative: Plougmann & Vingtoft A/S

(57) **Abstract**

The present invention relates to a microarray for detection of fusion genes comprising a chimeric probe for an exon-to-exon junction of a fusion gene. The microarray can be used for detection of fusion genes, e.g. in relation to cancer. The invention also relates to a method for detection of fusion genes and to a kit comprising a microarray for detection of fusion arrays.

## Description

### Background

Cancer genomes often contain fusion genes, created after structural chromosomal rearrangements such as translocations, deletions, and inversion. Fusion genes are typically found in haematological cancers. So far, fusion genes have been found only rarely associated with solid tumours, in contrast to detection of numerous genomic copy number imbalances. However, recent reports have shown that fusion transcripts may prove to be a common contributor also to the development of solid tumours (Mitelman *et al.,* 2007, Teixeira, 2006, Tomlins *et al.,* 2005). The main problem has been the technological limitations for detection of fusion genes in solid tumours.

Identification of certain fusion genes are currently performed for differential diagnosis or therapeutic decision-making in haematological cancers and some rare solid tumour types. At present, routine diagnostics laboratories use laborious and inefficient analyses for detection of fusion genes in clinical samples. The tests are typically cytogenetic chromosome analyses (karyotyping - usually by Giemsa banding) and/or RT-PCR of a selection of the most common fusion genes covering the most common break points for the individual novel transcript. To obtain metaphase chromosomes for karyotyping, a considerable amount of fresh tissue material is required, which also need to contain living and dividing cells. This methodology is also time consuming and labour intensive, and yet only has a success rate of about 70 percent. Furthermore, it is necessary to have highly experienced and competent personnel to examine the chromosomes visually, providing subjective results that also are at low-resolution. RT-PCR is a focused method, enabling analysis of one or a few candidate fusion genes at the time, at pre-defined fusion break points within them. The major limitation of this method is that it is not genome-wide, and thus a negative finding is not conclusive.

### Background art

There have been a few reports trying to identify predetermined fusion genes by oligo microarrays targeting specific junction sequences. These relied on a preceding step with amplification of the probes by RT-PCR, specifically targeting a small selection of predefined fusion genes and individual junction sequences therein. Similarly, junction oligos between exons in the same gene have been used for detection of alternative splicing.

Nasedkina *et al.,* 2002, used multiplex RT-PCR followed by microarrays for identification of PCR products containing specific fusion transcripts. Their microarray contained probes for detection of up to two fusion variants of each of four well-known fusion genes. PCR amplification was performed as a nested two-round multiplex reaction with specific primers. Thus, their method and microarrays was designed for identification of only a few predetermined gene fusions.

Nasedkina *et al.,* 2003 expanded on the above findings to include probes targeting one additional fusion gene, and 247 cases of childhood leukaemia were screened. Again, the authors only aimed at identification of predetermined fusion genes, more specifically fusion genes of clinical relevance for childhood leukaemia.

Shi *et al.,* 2003 used multiplex RT-PCR for amplification of seven fusion genes and subsequently used oligo microarrays to identify the PCR product, i.e. oligos targeting one or two sites per fusion gene. As with Nasedkina *et al.,* 2002, Nasedkina *et al.,* 2003, their analysis was limited to a rather small number of predetermined fusion genes that are known to have an association with leukaemia. The authors claim that their method is quantitative, as opposed to the method of Nasedkina *et al.,* 2002, Nasedkina *et al.,* 2003. Further, Shi *et al.,* 2003 mention on page 1069 that *"Although multiplex RT-PCR with 10-20 primer pairs was ideal, our preliminary data indicated that multiplex RT-PCR with primer pairs in excess of 20 was achievable with substantial assay optimization effort. However, the probability that formation of non-specific PCR products and primer-dimers would increase with increasing numbers of primers limited the maximum number of primer pairs".* Thus, they acknowledge an unmet demand for higher throughput of the analysis and suggest that more than one multiplex RT-PCR can be devised to encompass more than 40 fusion transcripts. Further, the authors on page 1072 mention that *"Because some of the translocation fusion splice-junction sites may be a few kilobases distant from the 3' poly(A) tail on the mRNA, use of microarray assay alone is not possible at this stage because the reverse transcriptase is unable to generate cDNA long enough to reach the fusion splice-junction site".* In other words, sequence specific RT-PCR is necessary for the assay to function, which in turn limits the throughput of the method for the reasons mentioned above.

Use of oligo microarrays in the analysis of pre-mRNA splicing patterns have previously been described in for example Bingham *et al.,* 2006, Johnson *et al.,* 2003.

US 2006/0084105 describes a microarray comprising sets of probes for detection of gene products that are produced by pre-mRNA splicing of a selected gene. The array comprises 372 splice junctions within 64 genes.

US 2006/012952 and WO 03/014295 also relate to the use of microarrays for detection of pre-mRNA splice variants.

### Detailed description of the invention

### Brief description of the drawings

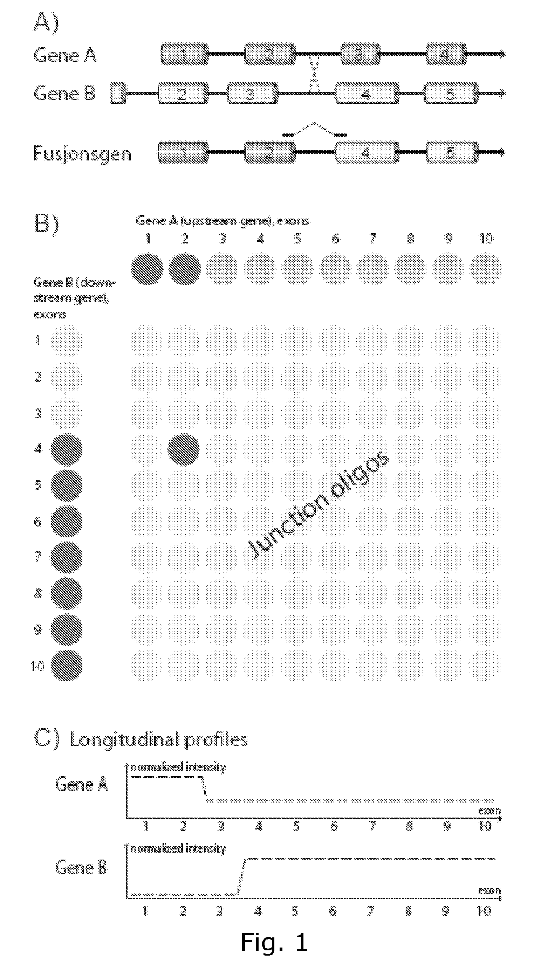

Figure 1. Microarray data pattern for a positive fusion gene hit. A) This hypothetical example of a fusion gene has a crossing over event between sequences in intron 2 in gene A and intron 3 in gene B. An intergenic exon-to-exon junction, A2-B4, oligo, detects the fusion transcript. B) If the genes A and B both have 10 exons, the microarray will contain 10 x 10 = 100 oligos to cover all exon-to-exon junction combinations for this particular fusion gene. The A2-B4 oligo detects the fusion transcript from part A. C) The longitudinal profiles of intragenic probes for each exon and exon-to-exon junction will provide support for true events of fusion genes.

### Summary of the invention

In a first aspect, the invention provides a microarray for detection of fusion genes comprising a chimeric probe for an exon-to-exon junction of a fusion gene. A second aspect of the invention is a method for detection of fusion genes and a third aspect of the invention is a kit comprising the microarray of the invention.

### Disclosure of the invention

A first aspect of the invention is a microarray for detection of fusion genes comprising a chimeric probe for an exon-to-exon junction of a fusion gene.

The fusion gene may be any fusion gene. Preferably, at least one of the fusion gene partners has previously been implicated as part of a verified fusion gene.

More preferably, the fusion gene is selected from the group consisting of the following known fusion genes,

**Table 1. Fusion genes, with the Ensembl gene IDs for each of the 275 pairs of fusion gene partners.**

| Gene_A | Gene_B |
|---|---|
| ENSG00000127914 | ENSG00000157764 |
| ENSG00000137727 | ENSG00000165288 |
| ENSG00000169696 | ENSG00000068323 |
| ENSG00000138363 | ENSG00000171094 |
| ENSG00000127152 | ENSG00000164438 |
| ENSG00000069399 | ENSG00000136997 |
| ENSG00000110987 | ENSG00000136997 |
| ENSG00000186716 | ENSG00000097007 |
| ENSG00000186716 | ENSG00000077782 |
| ENSG00000186716 | ENSG00000096968 |
| ENSG00000186716 | ENSG00000134853 |
| ENSG00000023445 | ENSG00000172175 |
| ENSG00000141867 | ENSG00000184507 |
| ENSG00000165288 | ENSG00000137727 |
| ENSG00000133639 | ENSG00000136997 |
| ENSG00000110619 | ENSG00000171094 |
| ENSG00000067955 | ENSG00000133392 |
| ENSG00000108091 | ENSG00000113721 |
| ENSG00000108091 | ENSG00000165731 |
| ENSG00000110092 | ENSG00000070404 |
| ENSG00000140937 | ENSG00000129204 |
| ENSG00000105810 | ENSG00000085276 |
| ENSG00000105810 | ENSG00000118058 |
| ENSG00000105810 | ENSG00000078403 |
| ENSG00000105810 | ENSG00000164438 |
| ENSG00000119397 | ENSG00000077782 |
| ENSG00000170791 | ENSG00000181690 |
| ENSG00000109220 | ENSG00000139083 |
| ENSG00000179583 | ENSG00000113916 |
| ENSG00000141367 | ENSG00000171094 |
| ENSG00000141367 | ENSG00000068323 |
| ENSG00000169714 | ENSG00000129204 |
| ENSG00000108821 | ENSG00000100311 |
| ENSG00000108821 | ENSG00000129204 |
| ENSG00000164692 | ENSG00000181690 |
| ENSG00000105662 | ENSG00000184384 |
| ENSG00000168036 | ENSG00000181690 |
| ENSG00000156976 | ENSG00000113916 |
| ENSG00000066629 | ENSG00000097007 |
| ENSG00000120616 | ENSG00000112511 |
| ENSG00000082805 | ENSG00000165731 |
| ENSG00000139083 | ENSG00000097007 |
| ENSG00000139083 | ENSG00000143322 |
| ENSG00000139083 | ENSG00000164398 |
| ENSG00000139083 | ENSG00000143437 |
| ENSG00000139083 | ENSG00000165556 |
| ENSG00000139083 | ENSG00000085276 |
| ENSG00000139083 | ENSG00000068078 |
| ENSG00000139083 | ENSG00000122025 |
| ENSG00000139083 | ENSG00000130675 |
| ENSG00000139083 | ENSG00000096968 |
| ENSG00000139083 | ENSG00000197880 |
| ENSG00000139083 | ENSG00000169184 |
| ENSG00000139083 | ENSG00000140538 |
| ENSG00000139083 | ENSG00000113721 |
| ENSG00000139083 | ENSG00000179094 |
| ENSG00000139083 | ENSG00000159216 |
| ENSG00000139083 | ENSG00000165025 |
| ENSG00000139083 | ENSG00000188580 |
| ENSG00000139083 | ENSG00000114999 |
| ENSG00000182944 | ENSG00000123268 |
| ENSG00000182944 | ENSG00000175197 |
| ENSG00000182944 | ENSG00000157554 |
| ENSG00000182944 | ENSG00000006468 |
| ENSG00000182944 | ENSG00000175832 |
| ENSG00000182944 | ENSG00000163497 |
| ENSG00000182944 | ENSG00000151702 |
| ENSG00000182944 | ENSG00000119508 |
| ENSG00000182944 | ENSG00000204531 |
| ENSG00000182944 | ENSG00000135605 |
| ENSG00000182944 | ENSG00000184937 |
| ENSG00000182944 | ENSG00000100105 |
| ENSG00000182944 | ENSG00000126746 |
| ENSG00000112486 | ENSG00000077782 |
| ENSG00000111790 | ENSG00000077782 |
| ENSG00000189283 | ENSG00000149948 |
| ENSG00000145216 | ENSG00000134853 |
| ENSG00000122025 | ENSG00000139083 |
| ENSG00000150907 | ENSG00000135903 |
| ENSG00000150907 | ENSG00000009709 |
| ENSG00000089280 | ENSG00000123268 |
| ENSG00000089280 | ENSG00000157613 |
| ENSG00000089280 | ENSG00000182158 |
| ENSG00000089280 | ENSG00000175197 |
| ENSG00000089280 | ENSG00000175197 |
| ENSG00000089280 | ENSG00000157554 |
| ENSG00000111640 | ENSG00000113916 |
| ENSG00000066455 | ENSG00000165731 |
| ENSG00000047932 | ENSG00000047936 |
| ENSG00000170961 | ENSG00000181690 |
| ENSG00000127946 | ENSG00000113721 |
| ENSG00000198339 | ENSG00000113916 |
| ENSG00000137309 | ENSG00000112769 |
| ENSG00000149948 | ENSG00000100814 |
| ENSG00000149948 | ENSG00000164919 |
| ENSG00000149948 | ENSG00000144476 |
| ENSG00000149948 | ENSG00000189283 |
| ENSG00000149948 | ENSG00000183722 |
| ENSG00000149948 | ENSG00000145012 |
| ENSG00000149948 | ENSG00000182185 |
| ENSG00000197711 | ENSG00000048544 |
| ENSG00000096384 | ENSG00000113916 |
| ENSG00000080824 | ENSG00000113916 |
| ENSG00000185811 | ENSG00000113916 |
| ENSG00000109471 | ENSG00000048462 |
| ENSG00000103522 | ENSG00000113916 |
| ENSG00000113263 | ENSG00000165025 |
| ENSG00000153814 | ENSG00000112511 |
| ENSG00000153814 | ENSG00000178691 |
| ENSG00000015133 | ENSG00000134853 |
| ENSG00000180843 | ENSG00000171094 |
| ENSG00000126777 | ENSG00000165731 |
| ENSG00000136167 | ENSG00000113916 |
| ENSG00000113594 | ENSG00000181690 |
| ENSG00000204691 | ENSG00000112561 |
| ENSG00000116604 | ENSG00000071626 |
| ENSG00000118058 | ENSG00000136754 |
| ENSG00000118058 | ENSG00000172493 |
| ENSG00000118058 | ENSG00000144218 |
| ENSG00000118058 | ENSG00000072364 |
| ENSG00000118058 | ENSG00000145819 |
| ENSG00000118058 | ENSG00000196914 |
| ENSG00000118058 | ENSG00000137812 |
| ENSG00000118058 | ENSG00000110395 |
| ENSG00000118058 | ENSG00000172409 |
| ENSG00000118058 | ENSG00000005339 |
| ENSG00000118058 | ENSG00000138336 |
| ENSG00000118058 | ENSG00000136848 |
| ENSG00000118058 | ENSG00000105656 |
| ENSG00000118058 | ENSG00000100393 |
| ENSG00000118058 | ENSG00000085832 |
| ENSG00000118058 | ENSG00000187239 |
| ENSG00000118058 | ENSG00000118689 |
| ENSG00000118058 | ENSG00000007237 |
| ENSG00000118058 | ENSG00000163655 |
| ENSG00000118058 | ENSG00000171723 |
| ENSG00000118058 | ENSG00000002834 |
| ENSG00000118058 | ENSG00000145012 |
| ENSG00000118058 | ENSG00000101367 |
| ENSG00000118058 | ENSG00000118058 |
| ENSG00000118058 | ENSG00000130382 |
| ENSG00000118058 | ENSG00000078403 |
| ENSG00000118058 | ENSG00000143443 |
| ENSG00000118058 | ENSG00000171843 |
| ENSG00000118058 | ENSG00000130396 |
| ENSG00000118058 | ENSG00000108292 |
| ENSG00000118058 | ENSG00000184481 |
| ENSG00000118058 | ENSG00000142347 |
| ENSG00000118058 | ENSG00000008300 |
| ENSG00000118058 | ENSG00000073921 |
| ENSG00000118058 | ENSG00000131759 |
| ENSG00000118058 | ENSG00000079102 |
| ENSG00000118058 | ENSG00000168385 |
| ENSG00000118058 | ENSG00000184702 |
| ENSG00000118058 | ENSG00000125354 |
| ENSG00000118058 | ENSG00000184640 |
| ENSG00000118058 | ENSG00000138758 |
| ENSG00000118058 | ENSG00000141985 |
| ENSG00000118058 | ENSG00000154556 |
| ENSG00000118058 | ENSG00000166140 |
| ENSG00000153944 | ENSG00000078399 |
| ENSG00000147065 | ENSG00000171094 |
| ENSG00000136997 | ENSG00000110987 |
| ENSG00000136997 | ENSG00000133639 |
| ENSG00000100345 | ENSG00000171094 |
| ENSG00000083168 | ENSG00000143970 |
| ENSG00000083168 | ENSG00000005339 |
| ENSG00000083168 | ENSG00000100393 |
| ENSG00000083168 | ENSG00000140396 |
| ENSG00000156650 | ENSG00000005339 |
| ENSG00000196531 | ENSG00000113916 |
| ENSG00000138293 | ENSG00000165731 |
| ENSG00000077150 | ENSG00000059377 |
| ENSG00000100503 | ENSG00000113721 |
| ENSG00000147140 | ENSG00000068323 |
| ENSG00000181163 | ENSG00000171094 |
| ENSG00000181163 | ENSG00000178053 |
| ENSG00000181163 | ENSG00000131759 |
| ENSG00000137497 | ENSG00000131759 |
| ENSG00000126883 | ENSG00000097007 |
| ENSG00000126883 | ENSG00000124795 |
| ENSG00000126883 | ENSG00000119335 |
| ENSG00000110713 | ENSG00000148700 |
| ENSG00000110713 | ENSG00000024862 |
| ENSG00000110713 | ENSG00000178105 |
| ENSG00000110713 | ENSG00000005073 |
| ENSG00000110713 | ENSG00000106031 |
| ENSG00000110713 | ENSG00000078399 |
| ENSG00000110713 | ENSG00000123388 |
| ENSG00000110713 | ENSG00000123364 |
| ENSG00000110713 | ENSG00000128713 |
| ENSG00000110713 | ENSG00000128714 |
| ENSG00000110713 | ENSG00000073614 |
| ENSG00000110713 | ENSG00000165671 |
| ENSG00000110713 | ENSG00000116132 |
| ENSG00000110713 | ENSG00000167157 |
| ENSG00000110713 | ENSG00000164985 |
| ENSG00000110713 | ENSG00000138698 |
| ENSG00000110713 | ENSG00000198900 |
| ENSG00000110713 | ENSG00000147548 |
| ENSG00000184507 | ENSG00000141867 |
| ENSG00000127083 | ENSG00000129204 |
| ENSG00000196092 | ENSG00000139083 |
| ENSG00000125618 | ENSG00000132170 |
| ENSG00000078674 | ENSG00000096968 |
| ENSG00000078674 | ENSG00000165731 |
| ENSG00000178104 | ENSG00000113721 |
| ENSG00000073921 | ENSG00000078403 |
| ENSG00000137193 | ENSG00000113916 |
| ENSG00000140464 | ENSG00000131759 |
| ENSG00000110777 | ENSG00000113916 |
| ENSG00000143294 | ENSG00000068323 |
| ENSG00000142611 | ENSG00000085276 |
| ENSG00000108946 | ENSG00000165731 |
| ENSG00000029725 | ENSG00000113721 |
| ENSG00000153201 | ENSG00000171094 |
| ENSG00000162775 | ENSG00000196588 |
| ENSG00000168421 | ENSG00000113916 |
| ENSG00000117000 | ENSG00000116990 |
| ENSG00000163902 | ENSG00000085276 |
| ENSG00000159216 | ENSG00000129993 |
| ENSG00000159216 | ENSG00000085276 |
| ENSG00000159216 | ENSG00000206115 |
| ENSG00000159216 | ENSG00000206115 |
| ENSG00000159216 | ENSG00000116251 |
| ENSG00000159216 | ENSG00000079102 |
| ENSG00000159216 | ENSG00000109686 |
| ENSG00000159216 | ENSG00000106346 |
| ENSG00000159216 | ENSG00000198492 |
| ENSG00000159216 | ENSG00000143373 |
| ENSG00000138674 | ENSG00000171094 |
| ENSG00000112701 | ENSG00000188580 |
| ENSG00000116560 | ENSG00000068323 |
| ENSG00000112081 | ENSG00000113916 |
| ENSG00000128487 | ENSG00000113721 |
| ENSG00000141380 | ENSG00000126752 |
| ENSG00000141380 | ENSG00000187754 |
| ENSG00000141380 | ENSG00000204645 |
| ENSG00000184402 | ENSG00000126752 |
| ENSG00000173757 | ENSG00000131759 |
| ENSG00000172660 | ENSG00000128656 |
| ENSG00000172660 | ENSG00000119508 |
| ENSG00000172660 | ENSG00000135605 |
| ENSG00000172660 | ENSG00000126746 |
| ENSG00000162367 | ENSG00000123473 |
| ENSG00000188580 | ENSG00000139083 |
| ENSG00000187735 | ENSG00000181690 |
| ENSG00000140262 | ENSG00000119508 |
| ENSG00000140262 | ENSG00000135605 |
| ENSG00000071564 | ENSG00000108924 |
| ENSG00000071564 | ENSG00000185630 |
| ENSG00000071564 | ENSG00000105619 |
| ENSG00000114354 | ENSG00000171094 |
| ENSG00000114354 | ENSG00000119508 |
| ENSG00000114354 | ENSG00000198400 |
| ENSG00000072274 | ENSG00000113916 |
| ENSG00000054118 | ENSG00000129204 |
| ENSG00000196535 | ENSG00000077782 |
| ENSG00000184012 | ENSG00000157554 |
| ENSG00000184012 | ENSG00000006468 |
| ENSG00000184012 | ENSG00000175832 |
| ENSG00000067369 | ENSG00000113721 |
| ENSG00000143549 | ENSG00000171094 |
| ENSG00000143549 | ENSG00000198400 |
| ENSG00000143549 | ENSG00000113721 |
| ENSG00000167460 | ENSG00000171094 |
| ENSG00000047410 | ENSG00000105976 |
| ENSG00000047410 | ENSG00000198400 |
| ENSG00000122779 | ENSG00000077782 |
| ENSG00000122779 | ENSG00000131759 |
| ENSG00000197323 | ENSG00000165731 |
| ENSG00000100815 | ENSG00000113721 |
| ENSG00000114999 | ENSG00000139083 |
| ENSG00000109906 | ENSG00000131759 |
| ENSG00000121741 | ENSG00000077782 |

wherein Gene A is the upstream fusion gene partner of the fusion gene and Gene B is the downstream fusion gene partner of the fusion gene.
A chimeric probe as used herein is a nucleic acid or a nucleic acid analogue, capable of sequence-specific base pairing, which comprises a first sequence corresponding to an exon of a first gene and a second sequence corresponding to an exon of a second gene. Importantly, the first gene is different from the second gene, i.e. the probe covers an intergenic exon-to-exon junction. The term exon-to-exon junction, as used in the present context, refers to an intergenic exon-to-exon junction. The chimeric probe may consist of or comprise non-natural nucleotides such as LNA monomers (locked nucleic acid monomers), INA monomers (intercalating nucleic acid monomers), or PNA monomers (peptide nucleic acid monomers).

The term fusion gene as used herein refers to the result of a genomic aberration, such as a chromosomal translocation, deletion, or inversion, bringing sequences from two different genes together. That is, the fusion gene comprises at least one exon of an upstream gene partner of the gene fusion and at least one exon of a downstream gene partner of the fusion gene.

Herein, the term fusion gene also refers to a hypothetical fusion gene that has not been experimentally verified.

A fusion gene partner as used herein refers to a gene that donates at least one exon to a fusion gene. The exon(s) of an upstream fusion gene partner are placed upstream of the exon(s) of the other fusion gene partner in the fusion gene transcript, and *vice versa.*

Of particular interest for the present invention are fusion gene partners and fusion genes that have previously been implicated a role in cancer. Table 1 lists preferred fusion genes with Gene A being the upstream fusion gene partner of the fusion gene and Gene B being the downstream fusion gene partner of the fusion gene.

The vast majority of fusion gene partners are fused within intron regions to create the fusion gene (Novo *et al.,* 2007), and splicing of the pre-mRNA fusion transcript will connect exons creating an intergenic exon-to-exon junction in the fusion transcript.

Hypothetical intergenic exon-to-exon junctions can be predicted when the exon-intron structures of two fusion gene partners of a hypothetical fusion gene are known. Exons of the potential fusion gene partners can be retrieved from various internet-based genome databases, such as www.biomart.org.

In a preferred embodiment, the microarray of the invention comprises a chimeric probe for at least 20% of all possible exon-to-exon junctions of a fusion gene.

In another preferred embodiment, the microarray of the invention comprises a chimeric probe for at least 30% of all possible exon-to-exon junctions, such as at least 40%, at least 50%, at least 60%, at least 70%, at least 80%, or at least 90%.

In yet another embodiment, the microarray of the invention comprises chimeric probes for at least 20 exon-to-exon junctions of the fusion gene.

In still another preferred embodiment, the microarray comprises chimeric probes for at least 30 exon-to-exon junctions, at least 40 exon-to-exon junctions, at least 50 exon-to-exon junctions, at least 60 exon-to-exon junctions, at least 70 exon-to-exon junctions, at least 80 exon-to-exon junctions, such as at least 100 exon-to-exon junctions.

The present inventors have recognized that it may not be sufficient to test for previously characterized (experimentally verified) fusion genes with a pre-determined exon-to-exon junction and that it is desirable to test all possible exon-to-exon junctions of a particular fusion gene. Very often, the exact location of the exon-to-exon junction is not the decisive factor in determining whether a fusion gene is oncogenic or otherwise involved in or predictive of cancer or other conditions.

For example, for the *TMPRSS2-ERG* fusion gene, newly identified in prostate cancer (Tomlins *et al.,* 2005), fusion transcripts have already been determined with junctions after exons 1, 2, 3, 4, and 5 in *TMPRSS2,* and before exons 2, 3, 4, 5, and 6 in *ERG,* at many different combinations (Clark *et al.,* 2006). Thus, choosing the one or few junctions that are most prevalent, would give a considerable probability of false negative results. This particular fusion gene is also an example of a fusion gene being created by deletion of a relatively small chromosomal fragment (3 Mbp), subsequently joining the two fusion gene partners. This small aberration is invisible by cytogenetic analyses due to the resolution level.

Oncogenicity may simply lie in overexpression of the downstream part of the fusion gene. Therefore, one advantage of the present invention is that it does not rely on a single or few pre-determined exon-to-exon junctions, but it is capable of detecting all possible exon-to-exon junctions of a given fusion gene.

Another advantage is that the invention does not require fresh cells as do e.g. karyotyping, described in the background section. Moreover, interpreting the results of the microarray analysis is more straightforward than interpreting the result of karyotyping, which takes highly trained personnel. In principle, the set of intergenic exon-to-exon junction oligos on the microarray will only produce a significant signal at a spot corresponding to an exon-to-exon junctions present in a fusion gene transcript.

Further, in contrast to a cytogenetic approach, there is no risk for selection among cells with the current invention, because RNA from all the cells of the biological sample is included into the measurements.

In a preferred embodiment, the microarray of the invention comprises a chimeric probe for each possible exon-to-exon junction of the fusion gene.

Preferably, the microarray of the invention comprises chimeric probes for more than one fusion gene. Thus, in a preferred embodiment, the microarray of the invention comprises chimeric probes for a number of fusion genes listed in Table 1, selected from the group consisting of at least 5 fusion genes, at least 10 fusion genes, at least 20 fusion genes, at least 30 fusion genes, at least 40 fusion genes, at least 50 fusion genes, at least 75 fusion genes, at least 100 fusion genes, at least 150 fusion genes, at least 200 fusion genes, at least 250 fusion genes and 275 fusion genes.

In an even more preferred embodiment, the microarray of the invention comprises chimeric probes for each possible intergenic exon-to-exon junction for a number of fusion genes listed in Table 1, selected from the group consisting of at least 5 fusion genes, at least 10 fusion genes, at least 20 fusion genes, at least 30 fusion genes, at least 40 fusion genes, at least 50 fusion genes, at least 75 fusion genes, at least 100 fusion genes, at least 150 fusion genes, at least 200 fusion genes, at least 250 fusion genes and 275 fusion genes.

Most preferably, the microarray of the invention comprises chimeric probes for each possible intergenic exon-to-exon junction for all fusion genes listed in Table 1. Such a microarray is useful for identification of fusion genes in any sample and requires no prior knowledge of pre-dispositions to particular fusion genes based on e.g. cancer type or patient history.

The sequence of the chimeric probes of the microarray comprise a first part and a second part, wherein the first part corresponds to the 3' end of an exon sequence of an upstream fusion gene partner and a second part corresponds to the 5' end of an exon sequence of a downstream fusion gene partner, wherein said chimeric probes are either antisense probes oriented to hybridise to mRNA or doublestranded cDNA, or sense probes being oriented to hybridise to cDNA of the fusion genes. Thus, the term "corresponds" as used in this context refers to either the same sequence or the complementary sequence.

The microarray may comprise both antisense and sense probes for each exon-to-exon junction, i.e. may be useful for hybridisation with both cDNA and mRNA or both strands of a PCR product.

Preferably, the chimeric probes are isothermic, i.e. they are adjusted in length to have a melting temperature (Tm value) that differs by at most 20 degrees Celsius when hybridised to a complementary DNA sequence under the conditions employed for hybridisation of the microarray. In other embodiments, the Tm values differ by at most 40 degrees Celsius 35 degrees, Celsius 30 degrees Celsius, 25 degrees Celsius, 15 degrees Celsius, and 10 degrees Celsius, respectively. Isothermic probes are favourable to enable good hybridisation conditions across the complete set of oligos on the microarray.

Moreover, the first part and the second part of the chimeric probes are preferably adjusted in length to have a Tm value that differs at most 10 degree Celsius under the conditions employed for hybridisation of the microarray. In other embodiments, the Tm values differ by at most 16 degrees Celsius, 14 degrees Celsius, 12 degrees Celsius, 8 degrees Celsius, 6 degrees and 4 degrees Celsius.

Adjustment of the Tm value of a probe or part of a probe may be achieved because the Tm value is dependent on the length and percentage of guanines and cytosines in the nucleotide sequence of the probe or part of the probe. It may be decided that the chimeric probes should have a Tm-value of e.g. about 68 degrees Celsius. As a start, the Tm value of a chimeric probe of 10 nucleotides for the first and the second part may be used. If the Tm value for this probe is below 68 degrees Celsius, nucleotides may be added in a balanced manner to both the first and the second part until the overall Tm value of the chimeric probe is about 68 degrees Celsius. Thus, if the first part comprises more A, T, or U nucleotides than the second part, more nucleotides will have to be added to the first part. The procedure is performed using an oligo design algorithm.

In a preferred embodiment of the invention, the Tm of the chimeric probes are above the temperature used for hybridisation and the Tm of upstream or/and downstream parts of the chimeric probes is below the temperature used for hybridisation.

The Tm value of the chimeric probe is preferably selected from the group consisting of more than 45 degrees Celsius, more than 50 degrees Celsius, more than 55 degrees Celsius, more than 60 degrees Celsius, more than 65 degrees Celsius, more than 70 degrees Celsius and more than 75 degrees Celsius.

The length of the chimeric probe is preferably selected from the group consisting of less than 60 nucleotides, less than 55 nucleotides, less than 50 nucleotides, less than 45 nucleotides, less than 40 nucleotides and less than 35 nucleotides.

In a preferred embodiment of the invention, the microarray further comprises intragenic probes corresponding to intra-exon sequences, exon-to-exon junctions, exon-intron junctions and intron-exon junctions of the individual fusion gene partners of the microarray. Such intragenic probes may be used to determine the expression level of fusion genes and/or fusion gene partners. In a preferred embodiment, intragenic probes are used in varying amounts or lengths in separate spots to facilitate quantification and comparison.

Preferably, the microarray comprises intragenic probes corresponding to all intra-exon sequences, exon-to-exon junctions, exon-intron junctions and intron-exon junctions of the individual fusion gene partners of the microarray.

Even more preferably, the microarray comprises 2, 3, 4, or 5 intragenic probes corresponding to each intra-exon sequences.

In another preferred embodiment, the microarray further comprises chimeric probes targeting single nucleotide polymorphic (SNP) variants of exon-to-exon junctions. Such SNPs can be retrieved from a genome database (such as www.biomart.org) for all fusion gene partners of table 1. Where SNPs are located within a sequence flanking an exon-to-exon junction, chimeric probes including each of the SNP variants are constructed. By including the polymorphic variants of exon-to-exon junctions, it is ensured that fusion genes are not missed due to mismatches between nucleotide sequences of chimeric probes and exon-to-exon junctions.

The microarray of the invention may be purchased from several manufacturers, e.g. Agilent, Illumina, and Nimblegen. Positive signals on the microarray are typically detected by measuring fluorescence or chemiluminescence, obtained from directly or indirectly labelled nucleotides of the mRNA or cDNA.

The scoring of the exon-to-exon junction oligos is relatively straightforward. This is because the majority of the thousands of spots will be negative, and only the features with positive exon-to-exon junction oligos produce a significant positive signal. Existence of a fusion gene, creating a positive signal from a chimeric probe, is supported by corresponding shifts in the normalized longitudinal expression level profiles created by the intragenic probes of the two fusion gene partners.

A second aspect of the invention is a method of detecting a fusion gene comprising the steps of
a. Providing a sample
b. Isolating RNA from the sample
c. Detecting exon-to-exon junctions of mRNAs from the sample using the microarray of the invention
d. Thereby identifying fusion genes present in the sample

The method may comprise preparation of cDNA using either oligo-dT priming or random primers, such as hexamers. In this embodiment, the exon-to-exon junction is detected on the cDNA level.

The sample may be any biological material, such as e.g. blood or bone marrow from a patient or person suspected having a cancer. Another example of a sample is tissue obtained from a solid tumour.

A third aspect of the invention is a kit comprising the microarray of the invention and random primers for cDNA synthesis and/or oligo-dT primers for cDNA synthesis. Preferably, the kit further comprises a reverse transcriptase and reagents necessary for cDNA synthesis.

### Examples

### Example 1.

### Creation of junction oligos and microarray

For generation of the junction oligos, we created a computer script (written in the programming language Python) that automatically processes public genome data. For all genes, and all their transcripts, the exon sequences were retrieved. We used the www.biomart.org internet portal. For each fusion gene combination, end sequences (the last 30 nucleotides) of all GeneA exons and start-sequences (30 nt) of all GeneB exons were joined at all combinations. Next, an oligo design algorithm was used to create oligos from each of these possible fusion gene exon-to-exon junctions. We have here used Tm optimally at 68 Celsius, and with equalized Tm from each side of the junction. In our example, we have generated exon-to-exon junction oligos ranging 33 to 46 nucleotides in length.

In this way, 47427 junction oligos was designed for 275 fusion genes.

To increase the sensitivity and specificity, longitudinal intragenic oligos were also designed. These are sets of oligos measuring expression levels along the transcripts for the individual fusion gene partners. Three oligos were generated targeting internally to each exon sequence, at the start, mid, and end, and oligos were also generated targeting the intragenic exon-to-exon junctions. Exon-to-intron junctions, and intron-to-exon junctions were also included as the pre-mRNA processing machinery may alter the splicing pattern following removal or introduction of cis-acting splicing regulatory sequences.

The designed oligos are synthesised onto a Nimblegen microarray, which currently can contain 2.1 million different oligo sequences per microarray.

### Example 2. The method/microarray in action

We are using total RNA depleted for ribosomal RNA as the input for cDNA synthesis. Random primers (hexamers) are used in the priming reaction of the first strand cDNA synthesis step. The cDNA is labelled by inclusion of Cy3 and Cy5-modified dNTPs.

When images from the microarray are measured, positive fusion genes are scored when we observe the following.
1. Significantly increased intensities for a fusion gene junction oligo is indicative of the presence of that particular fusion gene, with that particular intergenic exon-to-exon junction in the fusion transcript.
2. Additionally, we have longitudinal oligos for each of the included fusion partner genes. These include three intra-exon oligos per exon, and exon-to-exon junction oligos. Here, we will see a difference in the normalized general gene expression levels between up- and downstream parts of the transcripts for each of the two fusion gene partners. As we move from the 5' to the 3' end of these transcripts, we will observe a drop in the expression levels in the upstream fusion partner (GeneA), and an increase in the signals for the downstream partner (GeneB). These shifts in normalized expression levels should occur at intragenic positions that correspond to the positive intergenic junction oligo as described in point 1.

For an RNA sample with a fusion transcript, we will see the combination of 1 and 2 above (see fig. 1).

### References

Bingham J, Sudarsanam S, and Srinivasan S (2006). Profiling human phosphodiesterase genes and splice isoforms. Biochem. Biophys. Res Commun., 350(1): 25-32.
Clark J, Merson S, Jhavar S, Flohr P, Edwards S, Foster CS, Eeles R, Martin FL, Phillips DH, Crundwell M, Christmas T, Thompson A, Fisher C, Kovacs G, and Cooper CS (2006). Diversity of TMPRSS2-ERG fusion transcripts in the human prostate. Oncogene, [Epub ahead of print].
Johnson JM, Castle J, Garrett-Engele P, Kan Z, Loerch PM, Armour CD, Santos R, Schadt EE, Stoughton R, and Shoemaker DD (2003). Genome-wide survey of human alternative pre-mRNA splicing with exon junction microarrays. Science, 302(5653): 2141-2144.
Mitelman F, Johansson B, and Mertens F (2007). The impact of translocations and gene fusions on cancer causation. Nat. Rev. Cancer., 7(4): 233-245.
Nasedkina T, Domer P, Zharinov V, Hoberg J, Lysov Y, and Mirzabekov A (2002). Identification of chromosomal translocations in leukemias by hybridization with oligonucleotide microarrays. Haematologica., 87(4): 363-372.
Nasedkina TV, Zharinov VS, Isaeva EA, Mityaeva ON, Yurasov RN, Surzhikov SA, Turigin AY, Rubina AY, Karachunskii AI, Gartenhaus RB, and Mirzabekov AD (2003). Clinical screening of gene rearrangements in childhood leukemia by using a multiplex polymerase chain reaction-microarray approach. Clin. Cancer Res., 9(15) : 5620-5629.
Novo FJ, de Mendíbil IO, and Vizmanos JL (2007). TICdb: a collection of mapped translocation breakpoints in cancer. BMC Genomics, 8: 33.
Shi RZ, Morrissey JM, and Rowley JD (2003). Screening and quantification of multiple chromosome translocations in human leukemia. Clin. Chem., 49(7): 1066-1073.
Teixeira MR (2006). Recurrent fusion oncogenes in carcinomas. Critical Rev. Oncogen., 12(3-4): 257-271.
Tomlins SA, Rhodes DR, Perner S, Dhanasekaran SM, Mehra R, Sun XW, Varambally S, Cao X, Tchinda J, Kuefer R, Lee C, Montie JE, Shah RB, Pienta KJ, Rubin MA, and Chinnaiyan AM (2005). Recurrent fusion of TMPRSS2 and ETS transcription factor genes in prostate cancer. Science, 310(5748): 644-648.

## Claims

1. A microarray for detection of fusion genes comprising a chimeric probe for an intergenic exon-to-exon junction of a fusion gene.

2. The microarray of claim 1, wherein the fusion gene is selected from the group consisting of the following fusion genes,
| Gene_A | Gene_B |
|---|---|
| ENSG00000127914 | ENSG00000157764 |
| ENSG00000137727 | ENSG00000165288 |
| ENSG00000169696 | ENSG00000068323 |
| ENSG00000138363 | ENSG00000171094 |
| ENSG00000127152 | ENSG00000164438 |
| ENSG00000069399 | ENSG00000136997 |
| ENSG00000110987 | ENSG00000136997 |
| ENSG00000186716 | ENSG00000097007 |
| ENSG00000186716 | ENSG00000077782 |
| ENSG00000186716 | ENSG00000096968 |
| ENSG00000186716 | ENSG00000134853 |
| ENSG00000023445 | ENSG00000172175 |
| ENSG00000141867 | ENSG00000184507 |
| ENSG00000165288 | ENSG00000137727 |
| ENSG00000133639 | ENSG00000136997 |
| ENSG00000110619 | ENSG00000171094 |
| ENSG00000067955 | ENSG00000133392 |
| ENSG00000108091 | ENSG00000113721 |
| ENSG00000108091 | ENSG00000165731 |
| ENSG00000110092 | ENSG00000070404 |
| ENSG00000140937 | ENSG00000129204 |
| ENSG00000105810 | ENSG00000085276 |
| ENSG00000105810 | ENSG00000118058 |
| ENSG00000105810 | ENSG00000078403 |
| ENSG00000105810 | ENSG00000164438 |
| ENSG00000119397 | ENSG00000077782 |
| ENSG00000170791 | ENSG00000181690 |
| ENSG00000109220 | ENSG00000139083 |
| ENSG00000179583 | ENSG00000113916 |
| ENSG00000141367 | ENSG00000171094 |
| ENSG00000141367 | ENSG00000068323 |
| ENSG00000169714 | ENSG00000129204 |
| ENSG00000108821 | ENSG00000100311 |
| ENSG00000108821 | ENSG00000129204 |
| ENSG00000164692 | ENSG00000181690 |
| ENSG00000105662 | ENSG00000184384 |
| ENSG00000168036 | ENSG00000181690 |
| ENSG00000156976 | ENSG00000113916 |
| ENSG00000066629 | ENSG00000097007 |
| ENSG00000120616 | ENSG00000112511 |
| ENSG00000082805 | ENSG00000165731 |
| ENSG00000139083 | ENSG00000097007 |
| ENSG00000139083 | ENSG00000143322 |
| ENSG00000139083 | ENSG00000164398 |
| ENSG00000139083 | ENSG00000143437 |
| ENSG00000139083 | ENSG00000165556 |
| ENSG00000139083 | ENSG00000085276 |
| ENSG00000139083 | ENSG00000068078 |
| ENSG00000139083 | ENSG00000122025 |
| ENSG00000139083 | ENSG00000130675 |
| ENSG00000139083 | ENSG00000096968 |
| ENSG00000139083 | ENSG00000197880 |
| ENSG00000139083 | ENSG00000169184 |
| ENSG00000139083 | ENSG00000140538 |
| ENSG00000139083 | ENSG00000113721 |
| ENSG00000139083 | ENSG00000179094 |
| ENSG00000139083 | ENSG00000159216 |
| ENSG00000139083 | ENSG00000165025 |
| ENSG00000139083 | ENSG00000188580 |
| ENSG00000139083 | ENSG00000114999 |
| ENSG00000182944 | ENSG00000123268 |
| ENSG00000182944 | ENSG00000175197 |
| ENSG00000182944 | ENSG00000157554 |
| ENSG00000182944 | ENSG00000006468 |
| ENSG00000182944 | ENSG00000175832 |
| ENSG00000182944 | ENSG00000163497 |
| ENSG00000182944 | ENSG00000151702 |
| ENSG00000182944 | ENSG00000119508 |
| ENSG00000182944 | ENSG00000204531 |
| ENSG00000182944 | ENSG00000135605 |
| ENSG00000182944 | ENSG00000184937 |
| ENSG00000182944 | ENSG00000100105 |
| ENSG00000182944 | ENSG00000126746 |
| ENSG00000112486 | ENSG00000077782 |
| ENSG00000111790 | ENSG00000077782 |
| ENSG00000189283 | ENSG00000149948 |
| ENSG00000145216 | ENSG00000134853 |
| ENSG00000122025 | ENSG00000139083 |
| ENSG00000150907 | ENSG00000135903 |
| ENSG00000150907 | ENSG00000009709 |
| ENSG00000089280 | ENSG00000123268 |
| ENSG00000089280 | ENSG00000157613 |
| ENSG00000089280 | ENSG00000182158 |
| ENSG00000089280 | ENSG00000175197 |
| ENSG00000089280 | ENSG00000175197 |
| ENSG00000089280 | ENSG00000157554 |
| ENSG00000111640 | ENSG00000113916 |
| ENSG00000066455 | ENSG00000165731 |
| ENSG00000047932 | ENSG00000047936 |
| ENSG00000170961 | ENSG00000181690 |
| ENSG00000127946 | ENSG00000113721 |
| ENSG00000198339 | ENSG00000113916 |
| ENSG00000137309 | ENSG00000112769 |
| ENSG00000149948 | ENSG00000100814 |
| ENSG00000149948 | ENSG00000164919 |
| ENSG00000149948 | ENSG00000144476 |
| ENSG00000149948 | ENSG00000189283 |
| ENSG00000149948 | ENSG00000183722 |
| ENSG00000149948 | ENSG00000145012 |
| ENSG00000149948 | ENSG00000182185 |
| ENSG00000197711 | ENSG00000048544 |
| ENSG00000096384 | ENSG00000113916 |
| ENSG00000080824 | ENSG00000113916 |
| ENSG00000185811 | ENSG00000113916 |
| ENSG00000109471 | ENSG00000048462 |
| ENSG00000103522 | ENSG00000113916 |
| ENSG00000113263 | ENSG00000165025 |
| ENSG00000153814 | ENSG00000112511 |
| ENSG00000153814 | ENSG00000178691 |
| ENSG00000015133 | ENSG00000134853 |
| ENSG00000180843 | ENSG00000171094 |
| ENSG00000126777 | ENSG00000165731 |
| ENSG00000136167 | ENSG00000113916 |
| ENSG00000113594 | ENSG00000181690 |
| ENSG00000204691 | ENSG00000112561 |
| ENSG00000116604 | ENSG00000071626 |
| ENSG00000118058 | ENSG00000136754 |
| ENSG00000118058 | ENSG00000172493 |
| ENSG00000118058 | ENSG00000144218 |
| ENSG00000118058 | ENSG00000072364 |
| ENSG00000118058 | ENSG00000145819 |
| ENSG00000118058 | ENSG00000196914 |
| ENSG00000118058 | ENSG00000137812 |
| ENSG00000118058 | ENSG00000110395 |
| ENSG00000118058 | ENSG00000172409 |
| ENSG00000118058 | ENSG00000005339 |
| ENSG00000118058 | ENSG00000138336 |
| ENSG00000118058 | ENSG00000136848 |
| ENSG00000118058 | ENSG00000105656 |
| ENSG00000118058 | ENSG00000100393 |
| ENSG00000118058 | ENSG00000085832 |
| ENSG00000118058 | ENSG00000187239 |
| ENSG00000118058 | ENSG00000118689 |
| ENSG00000118058 | ENSG00000007237 |
| ENSG00000118058 | ENSG00000163655 |
| ENSG00000118058 | ENSG00000171723 |
| ENSG00000118058 | ENSG00000002834 |
| ENSG00000118058 | ENSG00000145012 |
| ENSG00000118058 | ENSG00000101367 |
| ENSG00000118058 | ENSG00000118058 |
| ENSG00000118058 | ENSG00000130382 |
| ENSG00000118058 | ENSG00000078403 |
| ENSG00000118058 | ENSG00000143443 |
| ENSG00000118058 | ENSG00000171843 |
| ENSG00000118058 | ENSG00000130396 |
| ENSG00000118058 | ENSG00000108292 |
| ENSG00000118058 | ENSG00000184481 |
| ENSG00000118058 | ENSG00000142347 |
| ENSG00000118058 | ENSG00000008300 |
| ENSG00000118058 | ENSG00000073921 |
| ENSG00000118058 | ENSG00000131759 |
| ENSG00000118058 | ENSG00000079102 |
| ENSG00000118058 | ENSG00000168385 |
| ENSG00000118058 | ENSG00000184702 |
| ENSG00000118058 | ENSG00000125354 |
| ENSG00000118058 | ENSG00000184640 |
| ENSG00000118058 | ENSG00000138758 |
| ENSG00000118058 | ENSG00000141985 |
| ENSG00000118058 | ENSG00000154556 |
| ENSG00000118058 | ENSG00000166140 |
| ENSG00000153944 | ENSG00000078399 |
| ENSG00000147065 | ENSG00000171094 |
| ENSG00000136997 | ENSG00000110987 |
| ENSG00000136997 | ENSG00000133639 |
| ENSG00000100345 | ENSG00000171094 |
| ENSG00000083168 | ENSG00000143970 |
| ENSG00000083168 | ENSG00000005339 |
| ENSG00000083168 | ENSG00000100393 |
| ENSG00000083168 | ENSG00000140396 |
| ENSG00000156650 | ENSG00000005339 |
| ENSG00000196531 | ENSG00000113916 |
| ENSG00000138293 | ENSG00000165731 |
| ENSG00000077150 | ENSG00000059377 |
| ENSG00000100503 | ENSG00000113721 |
| ENSG00000147140 | ENSG00000068323 |
| ENSG00000181163 | ENSG00000171094 |
| ENSG00000181163 | ENSG00000178053 |
| ENSG00000181163 | ENSG00000131759 |
| ENSG00000137497 | ENSG00000131759 |
| ENSG00000126883 | ENSG00000097007 |
| ENSG00000126883 | ENSG00000124795 |
| ENSG00000126883 | ENSG00000119335 |
| ENSG00000110713 | ENSG00000148700 |
| ENSG00000110713 | ENSG00000024862 |
| ENSG00000110713 | ENSG00000178105 |
| ENSG00000110713 | ENSG00000005073 |
| ENSG00000110713 | ENSG00000106031 |
| ENSG00000110713 | ENSG00000078399 |
| ENSG00000110713 | ENSG00000123388 |
| ENSG00000110713 | ENSG00000123364 |
| ENSG00000110713 | ENSG00000128713 |
| ENSG00000110713 | ENSG00000128714 |
| ENSG00000110713 | ENSG00000073614 |
| ENSG00000110713 | ENSG00000165671 |
| ENSG00000110713 | ENSG00000116132 |
| ENSG00000110713 | ENSG00000167157 |
| ENSG00000110713 | ENSG00000164985 |
| ENSG00000110713 | ENSG00000138698 |
| ENSG00000110713 | ENSG00000198900 |
| ENSG00000110713 | ENSG00000147548 |
| ENSG00000184507 | ENSG00000141867 |
| ENSG00000127083 | ENSG00000129204 |
| ENSG00000196092 | ENSG00000139083 |
| ENSG00000125618 | ENSG00000132170 |
| ENSG00000078674 | ENSG00000096968 |
| ENSG00000078674 | ENSG00000165731 |
| ENSG00000178104 | ENSG00000113721 |
| ENSG00000073921 | ENSG00000078403 |
| ENSG00000137193 | ENSG00000113916 |
| ENSG00000140464 | ENSG00000131759 |
| ENSG00000110777 | ENSG00000113916 |
| ENSG00000143294 | ENSG00000068323 |
| ENSG00000142611 | ENSG00000085276 |
| ENSG00000108946 | ENSG00000165731 |
| ENSG00000029725 | ENSG00000113721 |
| ENSG00000153201 | ENSG00000171094 |
| ENSG00000162775 | ENSG00000196588 |
| ENSG00000168421 | ENSG00000113916 |
| ENSG00000117000 | ENSG00000116990 |
| ENSG00000163902 | ENSG00000085276 |
| ENSG00000159216 | ENSG00000129993 |
| ENSG00000159216 | ENSG00000085276 |
| ENSG00000159216 | ENSG00000206115 |
| ENSG00000159216 | ENSG00000206115 |
| ENSG00000159216 | ENSG00000116251 |
| ENSG00000159216 | ENSG00000079102 |
| ENSG00000159216 | ENSG00000109686 |
| ENSG00000159216 | ENSG00000106346 |
| ENSG00000159216 | ENSG00000198492 |
| ENSG00000159216 | ENSG00000143373 |
| ENSG00000138674 | ENSG00000171094 |
| ENSG00000112701 | ENSG00000188580 |
| ENSG00000116560 | ENSG00000068323 |
| ENSG00000112081 | ENSG00000113916 |
| ENSG00000128487 | ENSG00000113721 |
| ENSG00000141380 | ENSG00000126752 |
| ENSG00000141380 | ENSG00000187754 |
| ENSG00000141380 | ENSG00000204645 |
| ENSG00000184402 | ENSG00000126752 |
| ENSG00000173757 | ENSG00000131759 |
| ENSG00000172660 | ENSG00000128656 |
| ENSG00000172660 | ENSG00000119508 |
| ENSG00000172660 | ENSG00000135605 |
| ENSG00000172660 | ENSG00000126746 |
| ENSG00000162367 | ENSG00000123473 |
| ENSG00000188580 | ENSG00000139083 |
| ENSG00000187735 | ENSG00000181690 |
| ENSG00000140262 | ENSG00000119508 |
| ENSG00000140262 | ENSG00000135605 |
| ENSG00000071564 | ENSG00000108924 |
| ENSG00000071564 | ENSG00000185630 |
| ENSG00000071564 | ENSG00000105619 |
| ENSG00000114354 | ENSG00000171094 |
| ENSG00000114354 | ENSG00000119508 |
| ENSG00000114354 | ENSG00000198400 |
| ENSG00000072274 | ENSG00000113916 |
| ENSG00000054118 | ENSG00000129204 |
| ENSG00000196535 | ENSG00000077782 |
| ENSG00000184012 | ENSG00000157554 |
| ENSG00000184012 | ENSG00000006468 |
| ENSG00000184012 | ENSG00000175832 |
| ENSG00000067369 | ENSG00000113721 |
| ENSG00000143549 | ENSG00000171094 |
| ENSG00000143549 | ENSG00000198400 |
| ENSG00000143549 | ENSG00000113721 |
| ENSG00000167460 | ENSG00000171094 |
| ENSG00000047410 | ENSG00000105976 |
| ENSG00000047410 | ENSG00000198400 |
| ENSG00000122779 | ENSG00000077782 |
| ENSG00000122779 | ENSG00000131759 |
| ENSG00000197323 | ENSG00000165731 |
| ENSG00000100815 | ENSG00000113721 |
| ENSG00000114999 | ENSG00000139083 |
| ENSG00000109906 | ENSG00000131759 |
| ENSG00000121741 | ENSG00000077782 |
wherein gene A is the upstream fusion gene partner of the fusion gene and gene B is the downstream fusion gene partner of the fusion gene.

3. The microarray of any of the preceding claims, comprising a chimeric probe for at least 20% of the possible intergenic exon-to-exon junctions of the fusion gene.

4. The microarray of any of claims 1 or 2 comprising a chimeric probe for at least 20 intergenic exon-to-exon junctions of the fusion gene.

5. The microarray of any of the preceding claims, comprising a chimeric probe for each possible intergenic exon-to-exon junction of the fusion gene.

6. The microarray of any of the preceding claims comprising chimeric probes for at least 20 fusion genes listed in claim 2.

7. The microarray of any of the preceding claims comprising chimeric probes for all fusion genes listed in claim 2.

8. The microarray of any of the preceding claims, wherein the chimeric probes comprise a first part and a second part, wherein the first part corresponds to the 3' end of an exon of an upstream fusion gene partner and a second part corresponds to 5' end of a downstream fusion gene partner, and wherein said chimeric probes are either antisense probes oriented to hybridise to mRNA or cDNA or sense probes being oriented to hybridise to cDNA of the fusion genes.

9. The microarray of any of the preceding claims, comprising both antisense and sense probes for each intergenic exon-to-exon junction.

10. The microarray of any of the preceding claims, wherein the chimeric probes are adjusted in length to have a Tm value that differs by at most 5 degrees Celsius.

11. The microarray of any of the preceding claims, wherein the first part and the second part of the chimeric probes are adjusted in length to have a Tm value that differs at most 5 degree Celsius.

12. The microarray of any of the preceding claims, wherein the Tm value of the chimeric probes are above the temperature used for hybridisation and wherein the Tm of upstream or downstream parts of the chimeric probes is below the temperature used for hybridisation.

13. The microarray of any of the preceding claims, further comprising intragenic probes corresponding to intra-exon sequences, exon-to-exon junctions, exon-intron junctions and intron-exon junctions of the fusion gene partners.

14. The microarray of any of the preceding claims further comprising chimeric probes targeting single nucleotide polymorphic (SNP) variants of exon-to-exon junctions.

15. A method of detecting a fusion gene comprising the steps of
a. Providing a sample
b. Isolating RNA from the sample
c. Detecting exon-to-exon junctions of mRNAs or cDNA from the sample using the microarray of any of claims 1-14
d. Thereby identifying fusion genes present in the sample

16. The method of claim 15 further comprising preparation of cDNA using either oligo-dT priming or random primers, such as random hexamers.

17. A kit comprising the microarray of any of claims 1-14 and random primers for cDNA synthesis and/or oligo-dT primers for cDNA synthesis.
